# EUROPEAN PATENT APPLICATION

(11) **EP 3 578 145 A1**
(43) Date of publication of application: **11.12.2019**
(21) Application number: 17894922.8
(22) Date of filing: 31.01.2017
(51) Int. Cl.: A61F 5/44, A61F 2/00

(54) **MALE URINARY INCONTINENCE PREVENTION DEVICE**

(71) Applicant: Endovision Co., Ltd., Daegu 42709 (KR)
(72) Inventor: JUNG, Min Ho, Daegu 42752 (KR)
(74) Representative: Viering, Jentschura & Partner mbB Patent- und Rechtsanwälte
(86) International application number: PCT/KR2017/001032
(87) International publication number: WO 2018/143487

(57) **Abstract**

The present invention relates to a male urinary incontinence prevention device, and the subject matter relates to a male urinary incontinence prevention device comprising: a body having an accommodation space in which the penis is accommodated; a urethral pressing unit formed in the accommodation space so as to press the urethral canal; and an adjustment means for adjusting the size of the accommodation space. Therefore, the present invention is very conveniently worn or removed by allowing the size of the accommodation space to be easily adjusted according to a user's body type when an adjustment button is pressed during use, and has an overall shape similar to that of the penis so as not to be exposed to the outside when worn, thereby improving the user's satisfaction and self-esteem.

## Description

### Technical Field

The present invention relates to a male urinary incontinence prevention device and, more particularly, to a male urinary incontinence prevention device allowing the size of an accommodation space to be easily adjusted using a control unit, so that attachment and detachment is convenient, and having an overall shape similar to that of a penis, so that the male urinary incontinence prevention device is not exposed externally when worn on the penis.

### Background Art

Urinary incontinence is a phenomenon of uncontrolled leakage of urine. Recently, the attack rates of urinary incontinence are increasing due to increases in the population of senior citizens caused by extended life expectancy. Types of urinary incontinence may include true urinary incontinence characterized by urine leakage occurring without any triggering factor, stress urinary incontinence characterized by urine leakage caused by increased intra-abdominal pressure, urge urinary incontinence characterized by the control of urination being lost when there is a feeling of needing to urinate, and overflow urinary incontinence characterized by the involuntary release of urine from an overfull urinary bladder.

In particular, as prostate diseases, such as enlarged prostate and prostate cancer, have been increased in along with increases in the population of senior citizens and westernization of eating, the attacking rate of urinary incontinence after treatment of these diseases ranges from 5% to 45%.

Male stress urinary incontinence (SUI), occurring as a result of sphincter dysfunction after treatment of prostate diseases as described above, may cause a lot of inconvenience to patients. Diapers, pads, condom catheters, or the like are used as efforts to help patients live daily lives.

Urinary incontinence prevention devices or urinary incontinence prevention apparatuses have been developed and used in order to prevent uncontrolled leakage of urine in daily lives. The assignee of the present application also filed a urinary incontinence prevention apparatus.

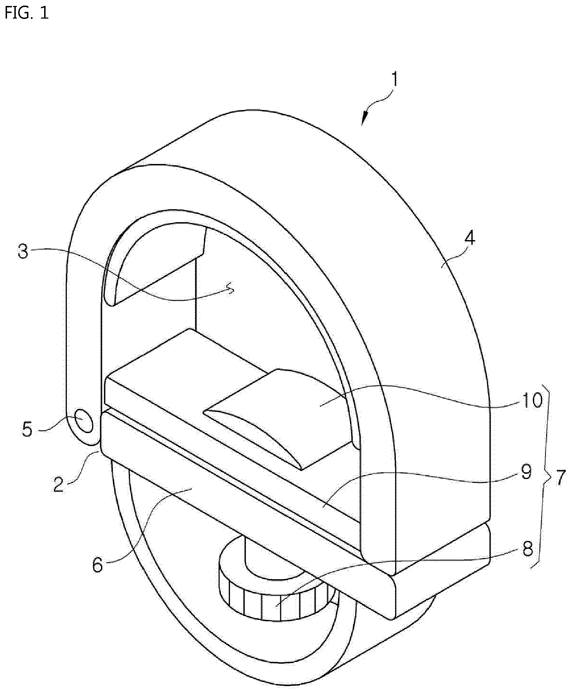

FIG. 1 illustrates a male urinary incontinence prevention device 1 of the related art. In the urinary incontinence prevention device 1, a body 4 has an open area 2 in one portion thereof, and an accommodation space 3 accommodating a penis is provided in a central portion of the body 4. A base 6 is detachably coupled to the open area 2 using a coupler 5. A urinary incontinence preventing unit 7 is provided on the central portion of the base 6 to prevent urinary incontinence by pressing the urethral canal of the penis.

The urinary incontinence preventing unit 7 includes a control member 8 fitted into a screw hole provided in the central portion of the base 6, a lift plate 9 coupled to a top end of the control member 8 to be moved up and down in the accommodation space 3 in response to the control member 8, and a compressor provided on the central portion of the top end of the control member 8 to press the urethral canal of the penis.

The related-art device is advantageous in that the urethral canal can be strongly pressed using the control member 8. However, the base 6 must be opened with respect to the body 4 before being worn on the penis and then be closed so as not to be separated from the penis, and the urinary incontinence preventing unit 7 must be moved upwards using the control member 8 to press the urethral canal. Accordingly, it is inconvenient for a patient to actually use the related-art device.

In addition, the size of the urinary incontinence prevention device is increased by an interference preventing member provided, in addition to the control member 8 protruding from the bottom surface of the body 4, to prevent interference between the control member 8 and clothing or the like. It is inconvenient to wear or use the urinary incontinence prevention device. In addition, even after the urinary incontinence prevention device is worn, the increased volume may cause the device to interfere with the body, which is problematic.

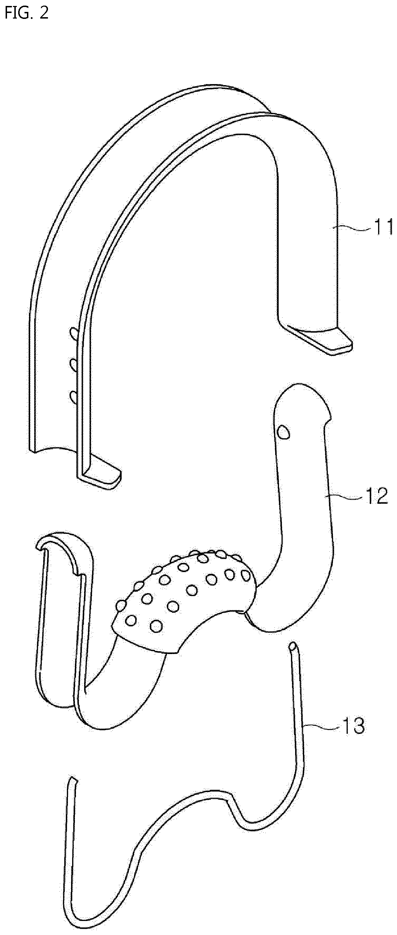

In order to overcome these problems, the assignee of the present application filed another male urinary incontinence prevention device, as illustrated in FIG. 2.

As illustrated in FIG. 2, the male urinary incontinence prevention device includes an upper member 11 located above a penis to hold the penis in position and a lower member 12 coupled to a bottom portion of the upper member 11. The lower member 12 prevents urinary incontinence by pressing the urethral canal of the penis while allowing the size of an accommodation space in which the penis is accommodated to be adjusted in a multi-stage manner.

This device has a simpler configuration than existing urinary incontinence prevention devices, so that a user can easily attach and detach the device. This device can minimize a foreign body sensation when worn on the user, and can be freely adjusted depending on the body shape of the user, thereby improving the convenience of use.

In addition, this device of the related art is provided with a separate elastic member 13 to improve the tightness of coupling between the upper member 11 and the lower member 12 and to hold the upper member 11 and the lower member 12 so that the upper member 11 and the lower member 12 are not separated from each other.

That is, the elastic member 13 conforms to the shape of the lower member 12 to press the lower member 12 inwards, thereby preventing the upper member 11 and the lower member 12 from being separated from each other.

The elastic member 13 is required to press the lower member 12 to couple the lower member 12 and the upper member 11, so that force is transferred to the upper member 11 while the lower member 12 freely moves along a contact surface between the lower member 12 and the upper member 11. However, it may not be easy to adjust elastic force using the elastic member 13, which is problematic.

In addition, the elastic member 13 may generate overall strong stress in the lower member 12 and the upper member 11, thereby disadvantageously increasing the risk of fracture even when a lower level of impact is applied.

In addition, when the lower member 12 is moved up and down in a position in which the upper member 11 and the lower member 12 are coupled by the elastic member 13, the lower member 12 is fixed in a specific position of the upper member 11. Accordingly, in a position in which the upper member 11 is fixed to specific position of a penis, it may not be easy to adjust the elastic member 13, which is problematic.

### Disclosure

### Technical Problem

Accordingly, the present invention has been made keeping in mind the above problems occurring in the prior art, and an object of the present invention is to provide a male urinary incontinence prevention device allowing the size of an accommodation space to be easily adjusted using a control unit, so that attachment and detachment is convenient, and having an overall shape similar to that of a penis, so that the male urinary incontinence prevention device is not exposed externally when worn on the penis.

### Technical Solution

In order to accomplish the above object, the present invention may provide a male urinary incontinence prevention device including: a body having an accommodation space in which a penis is accommodated; a urethra pressing unit provided in the accommodation space to press a urethral canal; and a control unit adjusting the size of the accommodation space.

In addition, the body may include: a first body; and a second body coupled to the first body to define the accommodation space.

In addition, the male urinary incontinence prevention device may further include a buffer pad provided within the body, in which the accommodation space may be adjusted in size by air injection or string tightening.

In addition, the urethra pressing unit may include a urethra pressing pad, in which the urethra pressing pad may be adjusted in size by air injection or string tightening.

In addition, the control unit may be one of a button type control unit, a pneumatic type control unit, a length adjustment type control unit, or a string adjustment type control unit.

Here, the button type control unit may be configured such that a portion of the body is expanded or contracted in response to an adjustment button provided on the body being manipulated, the height of the urethra pressing unit is adjusted in response to the adjustment button provided on the body being manipulated, or the first body and the second body are coupled to or decoupled from each other in a specific position in response to the adjustment button provided on the first body or the second body being manipulated.

In addition, the control unit may include: the adjustment button provided on the first body or the second body; and an adjustment bridge changing or fixing the position of the first body or the second body in response to the adjustment button being manipulated.

In addition, the adjustment button may include: a pressing portion coupled to a coupling hole provided in the first body to elastically move inward or outward of the first body; and an adjusting member provided on the pressing portion to prevent the pressing portion from being dislodged from the coupling hole.

In addition, the adjustment button may further include an elastic member coupled to the pressing portion to elastically move the pressing portion.

In addition, the adjustment bridge may include a plurality of engaging holes or a plurality of engaging lugs provided in the first body or the second body.

In addition, if the adjustment bridge includes the plurality of engaging holes, the adjustment button may be implemented as an elastic bridge extending from the pressing portion and coupled to the engaging holes.

In addition, if the adjustment bridge includes the plurality of engaging lugs, the adjustment bridge may include: a guide allowing the second body to move when the adjustment member elastically moves inwards in response to the adjustment button being manipulated; the plurality of engaging lugs provided on both sides of the guide to elastically move outwards to fix the second body in response to the pressing portion being released from a pressed position; and a guide path provided inward of the engaging lugs to communicate with the guide, the guide path being configured such that the adjustment member is received and seated therein when the pressing portion is pressed.

In addition, the adjustment member and the engaging lugs may be configured such abutting surfaces thereof are inclined in a corresponding manner.

In addition, the control unit may further include a support plate provided in a position facing the adjustment button to support an amount of pressure applied to the adjustment button.

In addition, the support plate may conform to a shape of the first body and is coupled to the first body, the support plate having an engaging portion to be fitted into a hole of the first body.

In addition, the support plate may be a buffer pad or may include a buffer pad provided therein. The buffer pad may be height adjusted by air injection or string tightening.

If the control unit is the air type control unit, the body may be provided as an air tube or include an air tube in a portion thereof, and the air type control unit may be operated by injecting air into the air tube.

In addition, if the control unit is the length adjustment type control unit, a portion of the body may be opened such that a bonding portion is provided on a leading end portion of the body, or a bonding portion may be provided on a coupling portion between the first body and the second body.

In addition, if the control unit is the string adjustment type control unit, a string, by which a portion of the body is drawn, may be provided, or a string, by which the first body or the second body is drawn, may be provided, and a tightening portion fixing the string in a specific position may be provided.

### Advantageous Effects

The present invention allows the size of the accommodation space to be easily adjusted using a control unit, so that attachment and detachment is convenient, and provides an overall shape similar to that of a penis, so that the male urinary incontinence prevention device is not exposed externally when worn on the penis. Accordingly, the satisfaction and self-respect of the user are improved.

In addition, the urethra pressing unit able to press the urethral canal is provided. Due to the control unit, the amount of pressure applied to the accommodation space, in which the urethra is accommodated, and the urethral canal can be easily adjusted. The device has a simple overall shape, minimizes damage in other portions of the human body due to interference between the other portions of the human body and clothing or the like, and facilitates attachment and detachment.

In addition, according to the present invention, the shape of the urethra pressing unit is improved to efficiently press the urethral canal, a foreign body sensation is minimized, and the urethra pressing area is maximized. Accordingly, a wearing sensation and the urinary incontinence prevention effect are advantageously improved.

Furthermore, the present invention allows a user to easily adjust the size of the accommodation space while pressing the adjustment button. The device can be manipulated in a simple manner and be used conveniently.

### Description of Drawings

FIGS. 1 and 2 are schematic views illustrating male urinary incontinence prevention devices of the related art;
FIG. 3 is a schematic view of the male urinary incontinence prevention device according to an embodiment of the present invention;
FIG. 4 is an exploded view of the male urinary incontinence prevention device according to the embodiment of the present invention;
FIG. 5 is a cross-sectional view of the male urinary incontinence prevention device according to the embodiment of the present invention;
FIG. 6 is an application view of the male urinary incontinence prevention device according to the embodiment of the present invention; and
FIGS. 7 to 11 are schematic views of a male urinary incontinence prevention device according to other embodiments of the present invention.

### Mode for Invention

The present invention relates to a male urinary incontinence prevention device. The male urinary incontinence prevention device allows the size of the accommodation space to be easily adjusted using a control unit, so that attachment and detachment is convenient, and provides an overall shape similar to that of a penis, so that the male urinary incontinence prevention device is not exposed externally when worn on the penis. Accordingly, the satisfaction and self-respect of the user are improved.

In addition, a urethra pressing unit able to press the urethral canal is provided. Due to the control unit, the amount of pressure applied to the accommodation space, in which the urethra is accommodated, and the urethral canal can be easily adjusted. The device has a simple overall shape, minimizes damage in other portions of the human body due to interference between the other portions of the human body and clothing or the like, and facilitates attachment and detachment.

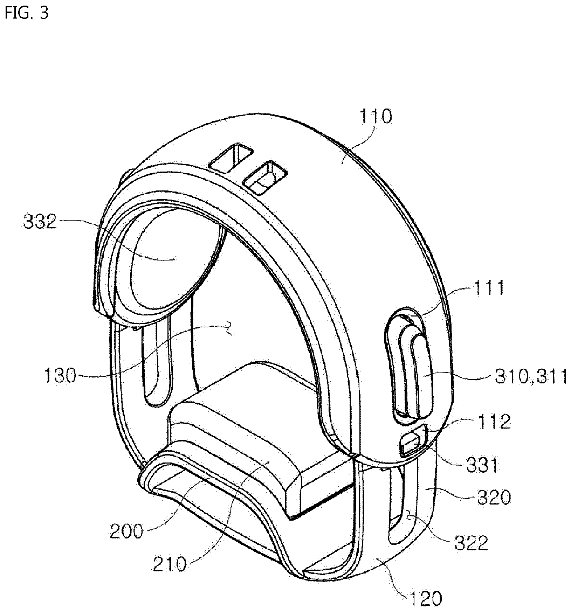

FIGS. 3 to 11 are schematic views illustrating a male urinary incontinence prevention device according to an embodiment of the present invention. The male urinary incontinence prevention device according to the present invention is characterized by including a body 100 having defined an accommodation space 130 in which a penis is accommodated, a urethra pressing unit 200 provided in the accommodation space 130 to press the urethra canal of the penis, and a control unit 300 adjusting the size of the accommodation space 130.

The body 100 has the accommodation space 130 in which a penis is accommodated. The entire components of the body 100 may be provided as a single member, or the body 100 may be comprised of a first body 110 and a second body 120 coupled to the first body 110 to define the accommodation space 130. For the sake of brevity, the first body 110 may refer to an upper body located above the penis, while the second body 120 may refer to a lower body located below the penis.

According to this configuration, a penis is accommodated in the accommodation space 130 within the body 100 or in a space between the first body 110 and the second body 120. In general, the upper portion of the penis is supported by a relatively wide area, while the lower portion of the penis is supported by a relatively narrow area, so that the urethra is selective pressed.

In addition, as required by a user, the size of the accommodation space 130 can be adjusted by contracting or expanding the frame of the body 100 using the control unit 300 or by adjusting the degree of coupling between the first body 110 and the second body 120.

In addition, the urethra pressing unit 200 is provided in a portion of the body 100, specifically, a lower portion of the accommodation space 130, to support a narrower area of the lower portion of the penis and selectively press the urethral canal.

That is, the urethra pressing unit 200 is provided in the lower portion of the accommodation space 130 in the body 100 or in the second body 120 forming the lower body. The urethra pressing unit 200 protrudes into the accommodation space 130 to support a narrow area of the penis and selectively press the urethral canal.

A buffer pad 332 may be further provided within the body 100. The buffer pad may conform to the shape of the body 100 while being made of a relatively-soft and flexible material, such as silicone, ethylene vinyl acetate (EVA), sponge, or rubber, in order to minimize pressure and friction applied to the penis.

In particular, the height of the buffer pad 332 may be adjusted depending on the body shape of the user by air injection or string tightening.

In addition, the urethra pressing unit 200 may further include a urethra pressing pad 210. The urethra pressing pad 210 may be made of a relatively-soft and flexible material, such as silicone, EVA, sponge, or rubber, in order to minimize pressure or friction applied to portions of the penis other than the urethral canal while minimizing a foreign body sensation and a wearing sensation when the urethral canal is pressed.

In addition, the height of the urethra pressing unit 200 may be adjusted by air injection or string tightening, so that the amount of pressure applied to the urethral canal can be adjusted depending on the body shape of the user.

In addition, the control unit 300 serves to adjust the size of the accommodation space 130 within the body 100 or the accommodation space 130 defined by the first body 110 and the second body 120, so that the accommodation space 130 is fitted to the body shape of the user. As described above, the control unit 300 serves to contact or expand a portion of the body 100 or adjust the level of coupling between the first body 110 and the second body 120. The control unit 300 may be provided in any manner as long as the user can conveniently manipulate the control unit 300 with one or both hands.

Specifically, a button may be provided on the body 100 to adjust the size of the accommodation space 130 by manipulating the button or by a one-touch method, an air pad may be provided on the body 100 to adjust the size of the accommodation space 130 in a pneumatic manner, Velcro tape may be provided on the body 100 to adjust the size of the accommodation space 130 by using the Velcro tape, or a string 360 and a tightening unit for fixing the string 360 may be provided on the body 100 to adjust the size of the accommodation space 130 in a string-tightening manner.

The control unit 300 may be provided on the body 100, may be provided on one of the first body 110 and the second body 120, or may be provided on the connecting portions of the first body 110 and the second body 120, depending on the convenience of wearing and manipulation, so that the size of the accommodation space 130 may be adjusted.

As described above, the present invention makes it possible to easily adjust the amount of pressure applied to the accommodation space 130, in which the urethra is accommodated, and the urethral canal by contracting or expanding a portion of the body 100 or adjusting the degree of coupling between the first body 110 and the second body 120. It is possible to minimize damage in other portions of the human body due to interference between the other portions of the human body and clothing or the like. In addition, attachment and detachment may be advantageously convenient, due to the easy manipulation.

Hereinafter, embodiments of the present invention will be described in detail with reference to the accompanying drawings.

### <First Embodiment>

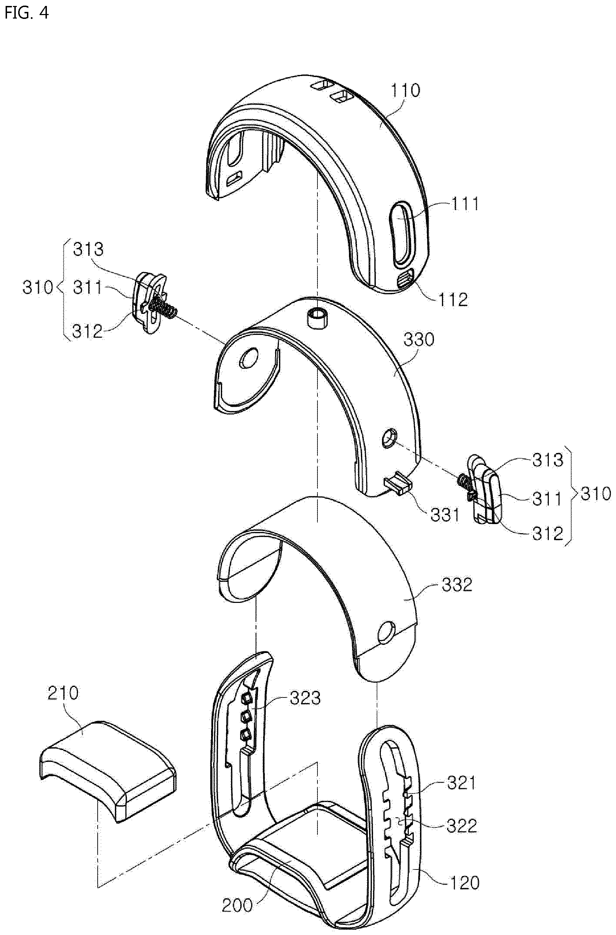

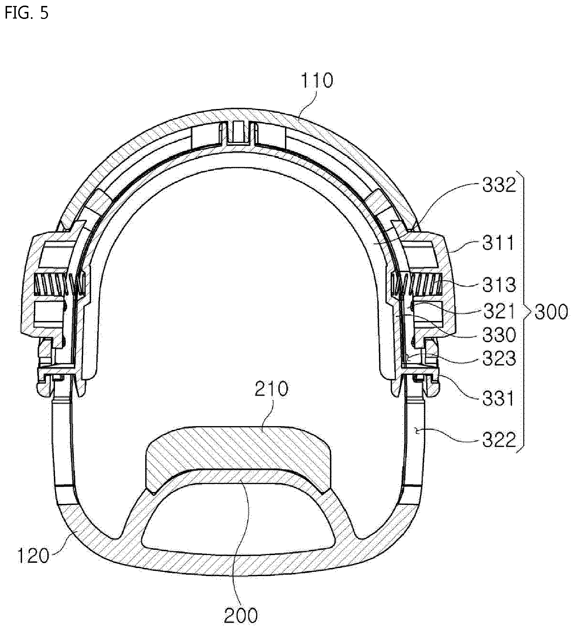

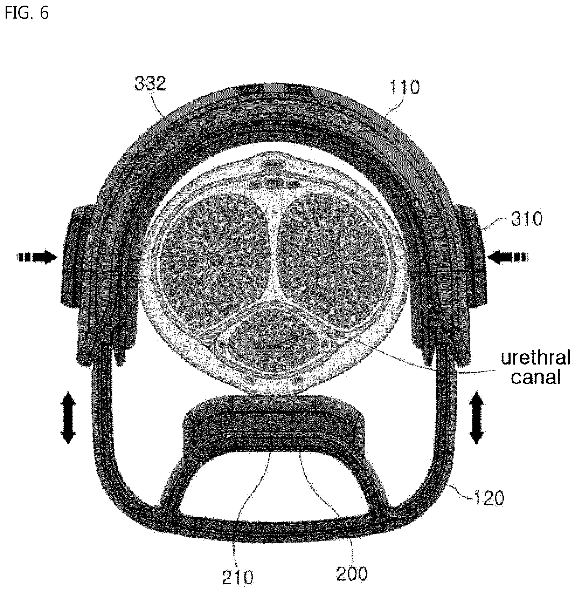

FIGS. 3 to 6 illustrate a first embodiment of the male urinary incontinence prevention device according to the present invention, in which FIG. 3 is a schematic view of the male urinary incontinence prevention device according to the embodiment of the present invention, FIG. 4 is an exploded view of the male urinary incontinence prevention device according to the embodiment of the present invention, FIG. 5 is a cross-sectional view of the male urinary incontinence prevention device according to the embodiment of the present invention, and FIG. 6 is an application view of the male urinary incontinence prevention device according to the embodiment of the present invention.

As illustrated in the figures, according to the first embodiment of the present invention, the control unit 300 is configured to be manipulated using the button. The first embodiment includes the first body 110, the second body 120 coupled to the bottom portion of the first body 110 to define the accommodation space 130 in which a penis is accommodated, the urethra pressing unit 200 provided on a portion of the body 120, in the direction of the accommodation space 130, so as to press the urethral canal, and the control unit 300 coupling the first body 110 and the second body 120 and allowing the second body 120 to be withdrawn from the first body 110 to adjust the size of the accommodation space 130.

That is, the penis is accommodated between the first body 110 and the second body 120, the urethra pressing unit 200 is provided on a portion of the second body 120 such that the urethra pressing unit 200 can press the urethral canal, and the second body 120 is configured to be withdrawn from the first body 110 by the control unit 300. The amount of pressure applied to the accommodation space 130, in which the urethra is accommodated, and the urethral canal can be easily adjusted. It is possible to minimize damage in other portions of the human body due to interference between the other portions of the human body and clothing or the like. In addition, attachment and detachment may be conveniently performed.

First, particularly, the first body 110 may have a general shape of an inverted U, with a central portion being rounded and both end portions being linear bridges, so as to conform to the shape of the penis and minimize interference and external exposure when worn.

That is, the first body 110 is configured such that a portion thereof in direct contact with the upper portion of the penis surrounds the surface of the penis. The first body 110 has a gently-curved general shape, with both end portions thereof being linear, so as to be coupled with the second body 120.

The second body 120, which will be described later, is coupled to the lower portion of the first body 110 by means of the control unit 300. The control unit 300 allows the second body 120 to be withdrawn from the first body 110, so that the size of the accommodation space 130, in which the penis is accommodated, is adjustable.

In addition, the buffer pad 332 may be further provided within the body 100. The buffer pad 332 serves to minimize pressure and friction applied to the penis. The buffer pad 332 may conform to the shape of the body 100 while being made of a relatively-soft and flexible material, such as silicone, EVA, sponge, or rubber.

In addition, the second body 120 is coupled to the lower portion of the first body 110 to define the accommodation space 130, in which the penis is accommodated.

The second body 120 is configured to be withdrawn from the first body 110, thereby allowing the size of the accommodation space 130 to be adjusted. This is realized by the control unit 300, which will be described later.

The accommodation space 130 serves to accommodate the penis therein. The size of the accommodation space 130 is adjustable by the control unit 300. Accordingly, this can minimize interference during attachment and detachment, so that the urinary incontinence prevention device according to the present invention can be conveniently attached and detached.

In addition, the amount of pressure applied to the urethral canal can be adjusted by adjusting the size of the accommodation space 130, depending on the symptom and the degree of progress of the urinary incontinence.

In addition, the urethra pressing unit 200 is provided on the second body 120, in the direction of the accommodation space 130, so as to press the urethral canal.

Compared to the first body 110 surrounding the entire upper portion of the penis, the urethra pressing unit 200 is configured to gently protrude in the direction of the accommodation space 130, and is gently curved to maximize the area by which the urethra is pressed in order to efficiently press the urethral canal and minimize a foreign body sensation.

The urethra pressing pad 210 may be further provided on the upper portion of the urethra pressing unit 200. The urethra pressing pad 210 serves to minimizing a foreign body sensation and a wearing sensation when the urethral canal is pressed. The urethra pressing pad 210 may be made of a relatively-soft and flexible material, such as EVA or sponge.

Specific locations of the first body 110 and the second body 120 are coupled to and decoupled from each other by means of the control unit 300. The first body 110 and the second body 120 are configured such that the second body 120 (or the first body 110) is withdrawn from or inserted into the first body 110 (or the second body 120) during coupling between the first body 110 and the second body 120, so that the size of the accommodation space 130 is easily adjustable.

The control unit 300 is a means for adjusting the size of the accommodation space 130 defined by the first body 110 and the second body 120. The control unit 300 can adjust the size of the accommodation space 130 so that the accommodation space 130 surrounds the penis of the user and adjust the amount of pressure applied to the urethral canal.

The size of the accommodation space 130 may be adjusted by a variety of methods. For example, the adjustment of the size of the accommodation space 130 may be performed by adjusting the extent of withdrawal of the second body 120 from the first body 110 using a button. An air pad provided within the accommodation space 130 defined by the first body 110 and the second body 120 may be used to adjust the size of the accommodation space 130 by an air pumping method. In addition, the first body 110 and the second body 120 may be coupled to each other using Velcro tape, and the first body 110 or the second body 120 may be adjusted using the Velcro tape to adjust the size of the accommodation space 130.

According to the embodiment, the control unit 300 includes adjustment buttons 310 elastically coupled to both end portions of the first body 110, a support plate 330 coupled to an inner portion of the first body 110 to support elastic pressure of the adjustment buttons 310, and adjustment bridges 320 integrally provided on both end portions of the second body 120 to move up and down through engagement, in response to the adjustment buttons 310 being manipulated.

That is, when the adjustment buttons 310 elastically coupled to the first body 110 are pressed, the adjustment bridges 320 provided integrally with the second body 120 can move up and down through engagement, thereby adjusting the size of the accommodation space 130. Accordingly, in response to the operation of pressing and releasing the adjustment buttons 310 with one hand, the adjustment bridges 320 can move up and down, so that the size of the accommodation space 130 can be easily adjusted.

The adjustment buttons 310 are elastically coupled to both end portions of the first body 110. The support plate 330 is configured such that portions thereof face or adjoin the adjustment buttons 310 to realize support force in directions opposite to an elastic pressure in order to support the elastic pressure of the adjustment buttons 310.

The support plate 330 is coupled to an inner portion of the first body 110. When the adjustment buttons 310 are pressed, elastic members 313 of the adjustment buttons 310 are in contact with and pressed to the adjoin portions of the support plate 330. Accordingly, support force is generated, causing elastic pressure toward the adjustment buttons 310.

The support plate 330 conforms to the shape of the first body 110. In position in which the support plate 330 adjoins the adjustment buttons 310, the support plate 330 and the first body 110 may be preferably spaced apart from each other by predetermined distances in order to provide play spaces in which the elastic members 313 are to be contracted and expanded to generate the elastic pressure of the adjustment buttons. That is, the linear bridges of the support plate 330 are more bent inward than the linear bridges of the first body 110.

In addition, the support plate 330 conforms to the shape of the first body 110, and is coupled to the inner portion of the first body 110. Engaging portions 331 are provided on both end portions of the support plate 330 to be fitted into holes 112 of the first body 110 in order to support the elastic pressure of the adjustment buttons 310.

That is, the support plate 330 is coupled to the inner portion of the first body 110. A coupling method may preferably be interference fitting performed using protrusions and recesses formed in corresponding positions. The engaging portions 331 are provided on both end portions of the support plate 330 to be fitted into the holes 112 of the first body 110.

The engaging portions 331 serve to fix the positions of the support plate 330 and the first body 110 for providing the play spaces in which the elastic members 313 for generating the elastic pressure of the adjustment buttons 310 are contracted or expanded. The engaging portions 331 are caught by the holes 112 so that the support plate 330 is not pushed inwards even if the support plate 330 is excessively pressed by the adjustment buttons 310, thereby limiting the elastic pressure.

In addition, the support plate 330 may be provided as a buffer pad by itself, or the buffer pad 332 may be further provided on the inner portion of the support plate 330. That is, the support plate 330 provided on the inner portion of the first body 110 may be provided as a buffer pad by itself. Alternatively, in a situation in which the support plate 330 is present, the buffer pad 332 may be further provided on the inner portion of the support plate 330.

The buffer pad 332 serves to minimize pressure or friction applied to the other portions of the penis except for the urethral canal, as described above. The buffer pad 332 conforms the shape of the first body 110, and may be made of a relatively-soft material, such as silicone, EVA, sponge, or rubber.

In addition, the adjustment bridges 320 are integrally provided on both end portions of the second body 120, that is, the linear bridges of the second body 120. The adjustment bridges 320 are coupled to the linear bridge areas of the first body 110, thereby allowing the second body 120 to move up and down in response to the adjustment buttons 310 being manipulated.

Here, the adjustment buttons 310 are elastically coupled to both end portions of the first body 110. Specifically, each of the adjustment buttons 310 includes a pressing portion 311 coupled to a coupling hole 111 of the first body 110 to be supported by the support plate 330 so as to elastically move inward and outward of the first body 110, adjustment members 312 provided on both surfaces of the pressing portion 311 to prevent the pressing portion 311 from being dislodged outward of the coupling hole 111, and the elastic member 313 coupled to an inner portion of the pressing portion 311 to elastically move the pressing portion 311 while elastically pressing one portion of the support plate 330.

The pressing portion 311 of the adjustment button 310 is provided to be exposed externally from both end portions of the first body 110, i.e. exposed externally from the linear bridges, such that pressing portion 311 is elastically movable inward and outward of the first body 110 when pressing is applied or removed.

The pressing portion 311 is coupled to the coupling hole 111 of the first body 110. A portion of the area of the support plate 330 is exposed through the coupling hole 111, such that the support plate 330 supports the elastic pressure, in response to the pressing portion 311 being pressed.

The adjustment members 312 protrude from both surfaces of the pressing portion 311 to be caught within the coupling hole 111, thereby preventing the pressing portion 311 from being dislodged from the coupling hole 111.

In addition, when the pressing portion 311 is pressed, the adjustment member 312 is elastically moved inward. The adjustment member 312 is released from a fixed position with respect to engaging lugs 321 or engaging holes, which will be described later, so that second body 120 can move up and down. When the pressing portion 311 is released from the pressed position, the adjustment member 312 is elastically moved outward again, so that the adjustment member 312 is caught by one of the adjustment member 312 in a specific position and simultaneously caught by the coupling hole 111. Accordingly, the position of the second body 120, thereby adjusting the size of the accommodation space 130. The pressing portion 311 of the adjustment button 310 is caught by the coupling hole 111, so that the manipulation is stopped.

The elastic member 313 is coupled to the inner portions of the pressing portion 311 to elastically move the pressing portion 311 while elastically pressing one portion of the support plate 330. For example, the elastic member 313 is made of an elastically resilient material, such as spring material or a rubber material.

In addition, the adjustment bridges 320 of the control unit 300 are integrally provided on the linear bridges of the end portions of the second body 120. The adjustment bridges 320 are configured to move up and down through engagement, in response to the adjustment buttons 310, e.g. the pressing portions 311, being manipulated. Accordingly, it is possible to easily adjust the size of the accommodation space 130 and the amount of pressure applied to the urethral canal.

The adjustment bridges 320 may be preferably provided as the plurality of engaging holes or the plurality of engaging lugs 321 in the first body 110 or the second body 120.

In a situation in which the adjustment bridges 320 are provided as the plurality of engaging holes (not shown), the adjustment buttons 310 may be provided as elastic bridges extending from the pressing portions 311 to be coupled to the engaging holes. That is, when the pressing portions 311 are pressed, the elastic bridges may move to be coupled to or decoupled from the engaging holes, thereby allowing the first body 110 or the second body 120 to move. Similarly to the principle of operation of an automatic umbrella, when the pressing portions 311 are pressed, the elastic bridges coupled to the engaging holes are decoupled and move to decouple the first body 110 or the second body 120. When the pressing portions 311 are not pressed, the elastic bridges are coupled to the engaging holes, so that the first body 110 and the second body 120 are coupled and fixed.

In addition, in a situation in which each of the adjustment bridges 320 is comprised of the plurality of engaging lugs 321, each of the adjustment bridges 320 includes a guide 322, the plurality of engaging lugs 321, and a guide path 323. The guide 322 is provided on an either end portion of the second body 120 to allow the second body 120 to move up and down in response to the adjustment members 312 being elastically moved by pressure of the pressing portions 311. The plurality of engaging lugs 321 are provided on both sides of the guide 322. When the pressing portion 311 is released from a pressed position, the corresponding adjustment members 312 elastically move outwards, so that the second body 120 is fixed at a predetermined height. The guide path 323 is provided inward of the engaging lugs 321 to communicate with the guide 322. When the pressing portion 311 is pressed, the adjustment member 312 is received and seated in the guide path 323.

The guide 322 is formed by cutting a bridge portion of the second body 120. The guide 322 provides a path allowing the second body 120 to move up and down with respect to the adjustment button 310.

In addition, the plurality of engaging lugs 321 are provided on both sides of the guide 322. When the pressing portion 311 is pressed, the adjustment member 312 elastically moves inwards, and the adjustment member 312 is released from the position fixed to the engaging lugs 321, so that the second body 120 can move up and down. When the pressing portion 311 is released from the pressed position, the adjustment member 312 elastically moves outwards again, so that the adjustment member 312 is caught at a predetermined position of the engaging lugs 321. Consequently, the movement of the second body 120 is stopped at the predetermined position, thereby forming the accommodation space 130 having a predetermined size.

In addition, the guide path 323 is formed inward of the engaging lugs 321 to communicate with the guide 322. When the pressing portion 311 is pressed, the adjustment member 312 is received and seated within the engaging lugs 321 after being released from the engaging lugs 321, so that the second body 120 can move up and down in a situation in which the adjustment member 312 is released from the position caught by the engaging lugs 321.

Here, the adjustment member 312 and the engaging lugs 321 are preferably configured such that abutting surfaces of the adjustment member 312 and the engaging lugs 321 are inclined in a corresponding manner. Accordingly, the adjustment member 312 can be easily caught by and released from the engaging lugs 321, while the interference of the adjustment bridges 320 to the movement of the second body 120 is minimized.

### <Second Embodiment>

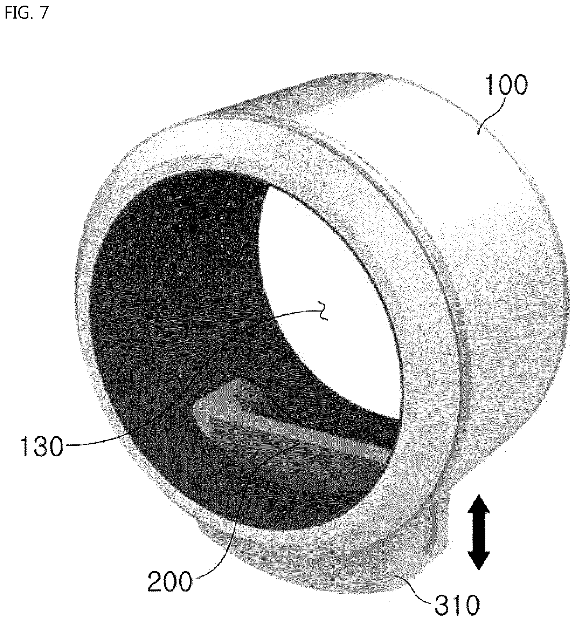

FIG. 7 is a schematic view illustrating a second embodiment of the male urinary incontinence prevention device according to the invention. In the second embodiment, descriptions of some features will be omitted when they are identical to those of the above-described first embodiment of the present invention.

As illustrated in the figure, according to the second embodiment of the present invention, the control unit 300 is manipulated using buttons in substantially the same manner as in the first embodiment. Differently from the first embodiment, in which the first body 110 and the second body 120 are provided separately, the body 100 is provided integrally. The control unit 300 is provided on a specific position of the body 100 as a single structure so as to be manipulated using buttons or switches, such that the size of the accommodation space 130 can be adjusted.

Specifically, the male urinary incontinence prevention device is configured such that the height of the urethra pressing unit 200 is adjusted in response to the adjustment buttons 310 of the body 100 being manipulated. The male urinary incontinence prevention device includes the body 100 in which the accommodation space 130 is defined, the urethra pressing unit 200 provided in a lower portion of the body 100 in the direction of the accommodation space 130 to press the urethral canal, and the control unit 300 able to adjust the size of the accommodation space 130, i.e. the height of urethra pressing unit 200, by moving the urethra pressing unit 200 toward the accommodation space 130.

That is, a penis is accommodated in the accommodation space 130 within the body 100. The urethra pressing unit 200 able to press the urethral canal is provided in the lower portion of the body 100. The height of the urethra pressing unit 200 is adjustable by the control unit 300. Accordingly, the amount of pressure applied to the accommodation space 130, in which the urethra is accommodated, and urethral canal can be easily adjusted. It is possible to minimize damage in other portions of the human body due to interference between the other portions of the human body and clothing or the like. In addition, attachment and detachment may be advantageously convenient.

The body 100 conforms to the shape of the penis, as described above. The body 100 may have a generally circular shape to surround the surface of the penis in order to minimize interference and external exposure when worn. The urethra pressing unit 200 protrudes in the accommodation space 130 to support the penis in a narrower area, thereby selectively pressing the urethral canal.

In addition, the control unit 300 is provided as a button type. When pressed upwards, the urethra pressing unit 200 is moved up and then fixed. When pressed once more, the fixed position is removed, so that the urethra pressing unit 200 moves downwards.

### <Third Embodiment>

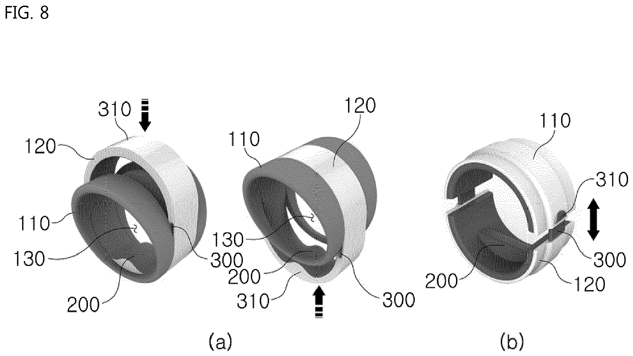

FIG. 8 is a schematic view illustrating a third embodiment of the male urinary incontinence prevention device according to the invention. In the third embodiment, descriptions of some features will be omitted when they are identical to those of the above-described first embodiment of the present invention.

As illustrated in the figure, according to the third embodiment of the present invention, the control unit 300 is manipulated using buttons in substantially the same manner as in the first embodiment. Here, the control unit 300 couples the first body 110 and the second body 120, while the first body 110 and the second body 120 are coupled to and decoupled from each other in a specific position, in response to the adjustment button 310 being manipulated.

Here, the control unit 300 is provided on connected portions of the first body 110 and the second body 120. When the first body 110 or the second body 120 is pressed, the height of the urethra pressing unit 200 provided in the first body 110 or the second body 120 is adjusted. The adjustment button 310 according to the third embodiment is implemented using the first body 110 or the second body 120.

Specifically, the height of the urethra pressing unit 200 is adjusted in response to the adjustment button 310, provided integrally with the first body 110 or the second body 120, being manipulated. The accommodation space 130 is defined by coupling between the first body 110 and the second body 120. The urethra pressing unit 200 is provided in the lower portion of the first body 110 or the second body 120, in the direction of the accommodation space 130, to press the urethral canal. The control unit 300 can adjust the size of the accommodation space 130, i.e. the height of the urethra pressing unit 200, by moving the urethra pressing unit 200 toward the accommodation space 130.

That is, the penis is accommodated in the accommodation space 130 defined by the first body 110 and the second body 120. The urethra pressing unit 200 able to press the urethral canal is provided in the lower portion of the first body 110 or the second body 120. The height of the urethra pressing unit 200 is adjusted by the control unit 300. The amount of pressure applied to the accommodation space 130, in which the urethra is accommodated, and urethral canal can be easily adjusted.

Referring to FIG. 8(a), in the male urinary incontinence prevention device including the control unit 300 according to the third embodiment of the invention, the first body 110 and the second body 120 are coupled to each other in an overlapping manner, and the first body 110 and the second body 120 are configured such that the size of the accommodation space 130 can be changed by pressing one of the first body 110 and the second body 120.

The coupling and decoupling of the first body 110 and the second body 120 are realized by the control unit 300. In particular, the adjustment button 310 may be implemented using the first body 110 or the second body 120 to adjust the size of the accommodation space 130, i.e. the height of the urethra pressing unit 200, by applying an amount of pressure thereto.

FIG. 8(b) illustrates another shape of the third embodiment of the present invention. A portion of the body is contracted or expanded in response to the adjustment button 310 being manipulated. The first body 110 and the second body 120 are disposed in the upper and lower portions, respectively. When the first body 110 or the second body 120 is pressed, the size of the accommodation space 130, i.e. the height of the urethra pressing unit 200, is changed. That is, the first body 110 and the second body 120 are coupled to and decoupled from each other in a specific position in response to the adjustment button 310 being manipulated, so that the size of the accommodation space 130 is changed.

### <Fourth Embodiment>

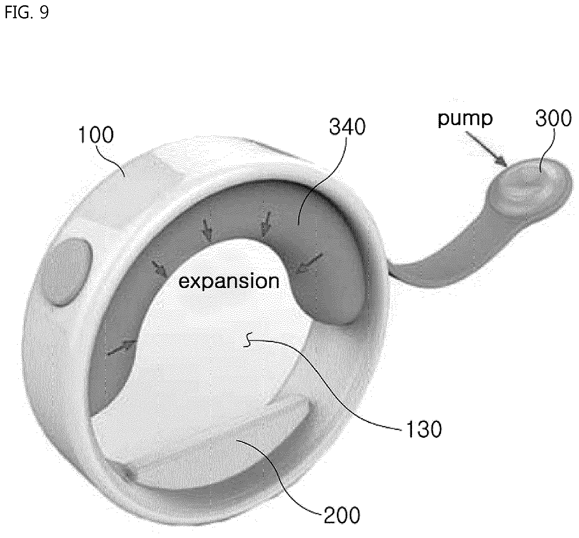

FIG. 9 is a schematic view illustrating a fourth embodiment of the male urinary incontinence prevention device according to the present invention. In the fourth embodiment, descriptions of some features will be omitted when they are identical to those of the above-described first embodiment of the present invention.

As illustrated in the figure, according to the fourth embodiment of the present invention, the control unit 300 is provided in a pneumatic manner. The body 100 may be implemented as the air tube 340 or the air tube 340 may be provided in the accommodation space 130 of the body 100. The size of the accommodation space 130 may be adjusted by expanding the air tube 340 by injecting air into the air tube 340 using a pump. In this case, the body 100 is a comprised of a single circular structure, and the air tube 340 may be provided in an upper portion or a portion corresponding to the urethra pressing unit 200, as required, such that the height can be adjusted by air injection.

### <Fifth Embodiment>

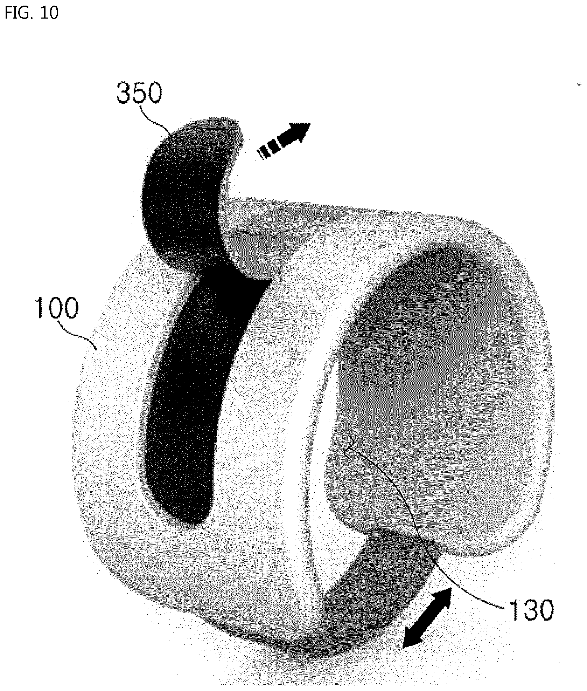

FIG. 10 is a schematic view illustrating a fifth embodiment of the male urinary incontinence prevention device according to the invention. In the fifth embodiment, descriptions of some features will be omitted when they are identical to those of the above-described first embodiment of the present invention.

As illustrated in the figure, according to the fifth embodiment of the present invention, the control unit 300 is length adjustable. A portion of the body 100 is opened, and a bonding portion 350 is provided in a leading end portion of the body 100. Alternatively, the bonding portion 350 may be provided in a coupling portion between the first body 110 and the second body 120.

Referring to FIG. 10, Velcro tape is provided on the leading end portion of an open portion of the body 100, such that the size of the accommodation space 130 is adjusted. Afterwards, the leading end portion of the body 100 is bonded to the body 100. The size of the accommodation space 130 is adjusted in this manner.

The shape of the urethra pressing unit 200 may be determined by employing the shape according to one of the first to fourth embodiments. Any shape may be used as long as the urethral canal can be pressed thereby.

### <Sixth Embodiment>

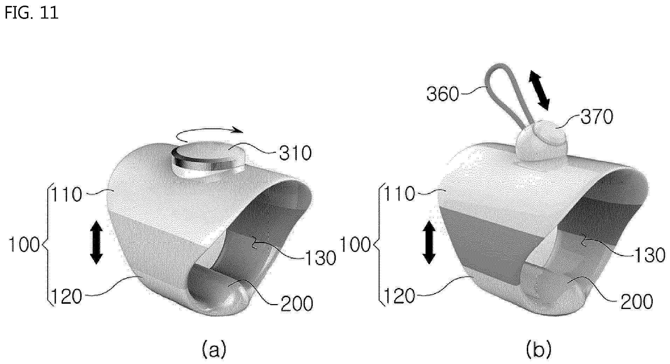

FIG. 11 is a schematic view illustrating a sixth embodiment of the male urinary incontinence prevention device according to the invention. In the sixth embodiment, descriptions of some features will be omitted when they are identical to those of the above-described first embodiment of the present invention.

As illustrated in the figure, according to the sixth embodiment of the present invention, the control unit 300 is provided in a string-tightening manner. The string 360, by which a portion of the body 100 is drawn, may be provided, or the string, by which the first body 110 or the second body 120 is drawn, may be provided. A tightening portion 370 for fixing the size of the accommodation space 130 by fixing the string 360 in a specific position may be provided.

Referring to FIG. 11(a), when the tightening portion 370 is turned, the string 360 may be wound into the tightening portion 370 so that the portion of the body 100 is folded, or the portion of the first body 110 or the second body 120 may be drawn so that the size of the accommodation space 130 is adjusted to be smaller. When the tightening portion 370 is turned in the opposite direction, the string 360 may be unwound so that the portion of the body is unfolded, or the first body 110 or the second body 120 may return to the original position so that the size of the accommodation space 130 is adjusted to be greater.

Referring to FIG. 11(b), when the string 360 is drawn, the portion of the body 100 may be drawn, or the portion of the first body 110 or the second body 120 may be drawn. The drawn string 360 may be fixed in a specific position using the tightening portion 370, so that the size of the accommodation space 130 may be adjusted.

Accordingly, the height of the urethra pressing unit 200 is automatically adjusted. The shape of the urethra pressing unit 200 may be determined by employing the shape according to one of the first to fourth embodiments. Any shape may be used as long as the urethral canal can be pressed thereby.

As set forth above, the male urinary incontinence prevention device according to the present invention allows the size of the accommodation space to be easily adjusted using a control unit, so that attachment and detachment is convenient, and provides an overall shape similar to that of a penis, so that the male urinary incontinence prevention device is not exposed externally when worn on the penis. Accordingly, the satisfaction and self-respect of the user are improved.

In addition, the urethra pressing unit able to press the urethral canal is provided. Due to the control unit, the amount of pressure applied to the accommodation space, in which the urethra is accommodated, and the urethral canal can be easily adjusted. The device has a simple overall shape, minimizes damage in other portions of the human body due to interference between the other portions of the human body and clothing or the like, and facilitates attachment and detachment.

In addition, according to the present invention, the shape of the urethra pressing unit is improved to efficiently press the urethral canal, a foreign body sensation is minimized, and the urethra pressing area is maximized. Accordingly, a wearing sensation and the urinary incontinence prevention effect are advantageously improved.

Furthermore, the present invention allows a user to easily adjust the size of the accommodation space while pressing the adjustment button. The device can be manipulated in a simple manner and be used conveniently.

## Claims

1. A male urinary incontinence prevention device comprising:
a body having an accommodation space in which a penis is accommodated;
a urethra pressing unit provided in the accommodation space to press a urethral canal; and
a control unit adjusting the size of the accommodation space.

2. The male urinary incontinence prevention device according to claim 1, wherein the body comprises:
a first body; and
a second body coupled to the first body to define the accommodation space.

3. The male urinary incontinence prevention device according to claim 1 or 2, further comprising a buffer pad provided within the body.

4. The male urinary incontinence prevention device according to claim 3, wherein the buffer pad is adjusted in height by air injection or string tightening.

5. The male urinary incontinence prevention device according to claim 1 or 2, wherein the urethra pressing unit comprises a urethra pressing pad.

6. The male urinary incontinence prevention device according to claim 5, wherein the urethra pressing pad is height adjusted by air injection or string tightening.

7. The male urinary incontinence prevention device according to claim 1 or 2, wherein the control unit is one of a button type control unit, a pneumatic type control unit, a length adjustment type control unit, or a string adjustment type control unit.

8. The male urinary incontinence prevention device according to claim 7, wherein the button type control unit is configured such that a portion of the body is expanded or contracted in response to an adjustment button provided on the body being manipulated, the height of the urethra pressing unit is adjusted in response to the adjustment button provided on the body being manipulated, or the first body and the second body are coupled to or decoupled from each other in a specific position in response to the adjustment button provided on the first body or the second body being manipulated.

9. The male urinary incontinence prevention device according to claim 8, wherein the control unit comprises:
the adjustment button provided on the first body or the second body; and
an adjustment bridge changing or fixing the position of the first body or the second body in response to the adjustment button being manipulated.

10. The male urinary incontinence prevention device according to claim 9, wherein the adjustment button comprises:
a pressing portion coupled to a coupling hole provided in the first body to elastically move inward or outward of the first body; and
an adjusting member provided on the pressing portion to prevent the pressing portion from being dislodged from the coupling hole.

11. The male urinary incontinence prevention device according to claim 10, wherein the adjustment button comprising an elastic member coupled to the pressing portion to elastically move the pressing portion.

12. The male urinary incontinence prevention device according to claim 10, wherein the adjustment bridge comprises a plurality of engaging holes or a plurality of engaging lugs provided in the first body or the second body.

13. The male urinary incontinence prevention device according to claim 12, wherein, if the adjustment bridge comprises the plurality of engaging holes, the adjustment button comprises an elastic bridge extending from the pressing portion and coupled to the engaging holes.

14. The male urinary incontinence prevention device according to claim 12, wherein, if the adjustment bridge comprises the plurality of engaging lugs, the adjustment bridge comprises:
a guide allowing the second body to move when the adjustment member elastically moves inwards in response to the adjustment button being manipulated;
the plurality of engaging lugs provided on both sides of the guide to elastically move outwards to fix the second body in response to the pressing portion being released from a pressed position; and
a guide path provided inward of the engaging lugs to communicate with the guide, the guide path being configured such that the adjustment member is received and seated therein when the pressing portion is pressed.

15. The male urinary incontinence prevention device according to claim 14, wherein the adjustment member and the engaging lugs are configured such abutting surfaces thereof are inclined in a corresponding manner.

16. The male urinary incontinence prevention device according to claim 8, wherein the control unit further comprises a support plate provided in a position facing the adjustment button to support an amount of pressure applied to the adjustment button.

17. The male urinary incontinence prevention device according to claim 16, wherein the support plate is provided to conform to a shape of the first body and is coupled to the first body, the support plate having an engaging portion to be fitted into a hole of the first body.

18. The male urinary incontinence prevention device according to claim 17, wherein the support plate is a buffer pad or further comprises a buffer pad provided therein.

19. The male urinary incontinence prevention device according to claim 18, wherein the buffer pad is height adjusted by air injection or string tightening.

20. The male urinary incontinence prevention device according to claim 7, wherein, if the control unit is the air type control unit, the body is provided as an air tube or includes an air tube in a portion thereof, and the air type control unit is operated by injecting air into the air tube.

21. The male urinary incontinence prevention device according to claim 7, wherein, if the control unit is the length adjustment type control unit, a portion of the body is opened such that a bonding portion is provided on a leading end portion of the body, or a bonding portion is provided on a coupling portion between the first body and the second body.

22. The male urinary incontinence prevention device according to claim 7, wherein, if the control unit is the string adjustment type control unit, a string, by which a portion of the body is drawn, is provided, or a string, by which the first body or the second body is drawn, is provided, and a tightening portion fixing the string in a specific position is provided.
